# EUROPEAN PATENT APPLICATION

(11) **EP 4 663 769 A2**
(43) Date of publication of application: **17.12.2025**
(21) Application number: 25171661.9
(22) Date of filing: 22.04.2025
(51) Int. Cl.: C12Q 1/6818, C12Q 1/6804

(54) **LABEL, MARKER AND METHOD FOR ANALYSING BIOLOGICAL SAMPLES**

(30) Priority: 21.05.2024 EP 24177155
(71) Applicant: Leica Microsystems CMS GmbH, 35578 Wetzlar (DE)
(72) Inventor: ALSHEIMER, Soeren, 35578 Wetzlar (DE)
(74) Representative: Schaumburg und Partner Patentanwälte mbB

(57) **Abstract**

A label (100, 200) for analysing a biological sample is provided. The label comprises a backbone comprising at least a first nucleic acid strand (102). The label further comprises at least one optically detectable labelling moiety (104) attached, in particular covalently attached, to the first nucleic acid strand (102). Additionally, the label comprises at least one hydrophilic moiety (106) attached, in particular covalently attached, to the backbone. In further aspects, a marker comprising the label (100, 200) is provided as well as a method for analysing a biological sample and a method to generate the label (100, 200).

## Description

### Technical field

The invention relates to a label for analysing biological samples comprising at least one labelling moiety and at least one hydrophilic moiety, as well as a corresponding marker. In further aspects, a method for detecting target analytes is disclosed as well as a method for generating the label.

### Background

Fluorescent markers are frequently used in bioassays due to their generally good sensitivity, specificity, and versatility in detecting and quantifying biological targets. These markers emit light upon excitation at specific wavelengths, allowing researchers to track molecular interactions, cellular events, or the presence of specific biomol-ecules. Fluorescent labeling can be applied to antibodies, nucleic acids, proteins, or small molecules, enabling a broad range of applications including flow cytometry, fluorescence microscopy, and ELISA-type assays. Their compatibility with multiplexing-using multiple fluorophores with distinct emission spectra-also allows simultaneous detection of several targets within a single sample.

It remains desirable to provide labels or markers that result in a minimal or no background noise when analysing biological samples. This is particularly the case when using large numbers of optically detectable labels or markers that need to be distinguished from each other.

### Summary

It is an object to provide a label and a marker that produce no background noise when analysing biological samples.

The aforementioned object is achieved by the subject-matter of the independent claims. Advantageous embodiments are defined in the dependent claims and the following description.

In a first aspect, a label for analysing a biological sample is provided. The label comprises a backbone comprising at least a first nucleic acid strand. The label further comprises at least one optically detectable labelling moiety attached, in particular covalently attached, to the first nucleic acid strand. Additionally, the label comprises at least one hydrophilic moiety attached, in particular covalently attached, to the backbone.

The label has several advantages, in particular due to its at least one hydrophilic moiety. For example, the label exhibits lower background (unspecific) binding, in particular, to random structures of the biological sample. In particular, this enables avoiding inadvertently labelling these random structures of the biological sample with the label.

Further, the label exhibits a reduced interaction with other molecules used to analyse the biological sample. For example, the label exhibits a reduced (unspecific) binding to affinity reagents, such as antibodies, that may be used to analyse the biological sample. This may reduce a detrimental effect of the label on the correct function of the affinity reagents.

Moreover, the label enables brighter light emission. For example, labelling moieties may tend to aggregate due to an attractive force between the labelling moieties. In particular, this may occur with hydrophobic labelling moieties. Aggregation of labelling moieties may cause a change of their native optical properties such as their emission intensity and/or absorbance spectra. This effect is particularly relevant with an increasing number of labelling moieties provided on a label or in close proximity to each other. This makes unambiguous identification of the labelling moieties difficult or impossible. Thus, the label enables reducing undesirable interactions between labelling moieties of the label and providing labels with labelling moieties that are easily identifiable based on their generally native optical properties.

Similarly, the interaction between several labels and their resulting aggregation may be reduced or avoided by providing the label with hydrophilic moieties. Moreover, when using affinity reagents such as antibodies when analysing biological samples, the label comprising the hydrophilic moieties may reduce or avoid unspecific interactions with these affinity reagents, which may otherwise result in a (partial) loss of function of these affinity reagents.

Overall, by reducing these undesired interactions or aggregations the label enables reducing or avoiding background noise when analysing biological samples. This is also achieved by enabling the labelling moieties of the label to maintain their native optical properties rather than a change in these native optical properties.

The backbone of the label may provide a structural framework, to which the at least one labelling moiety and the at least one hydrophilic moiety are attached. The backbone may be a polymeric backbone, in particular. Thus, the backbone may comprise repeating subunits. Preferably, the backbone is a biopolymer. The at least one labelling moiety and the at least one hydrophilic moiety may be attached to the subunits of the backbone.

The nucleic acid strands of the backbone may be synthetic nucleic acid strands, in particular. This means that the nucleic acid strands may be chemically synthesised, rather than be the product of a biochemical process of the biological sample to be analysed.

For example, the at least one optically detectable labelling moiety may be a fluorescent labelling moiety, the fluorescence of which may be optically detectable. In particular, the at least one optically detectable may be a fluorophore such as a fluorescent protein, a fluorescent small molecule, or a quantum dot.

The at least one optically detectable labelling moiety and/or the at least one hydrophilic moiety may be attached to the phosphate, pentose or nucleobase of nucleotides of one of the nucleic acid strands of the backbone of the label, in particular.

In particular, the hydrophilic moiety is not a nucleic acid and/or the hydrophilic moiety does not comprise a nucleotide. Further in particular, the hydrophilic moiety is not a labelling moiety, for example, the hydrophilic moiety does not (re-)emit light upon excitation.

For example, the hydrophilic moiety is configured to decrease the hydrophobicity of the backbone and/or the label. In particular, the hydrophilic moiety is hydrophilic under physiological conditions and/or in aqueous solution. Thus, the presence of the hydrophilic moiety causes the label to be hydrophilic, in particular. In a particular example, a measure of the hydrophobicity of the backbone and/or the label may be their polarity. In this regard it should be further noted that the phosphate backbone of DNA may be more polar than the hydrophilic moiety. However, the label also comprises the labelling moiety, which may be hydrophobic and the hydrophilic moiety, for example PEG groups, may shield these (hydrophobic) labelling moieties and thus have a net positive contribution towards hydrophilicity of the label. Similarly, the hydrophilic moiety may reduce non-specific binding of the label.

The biological sample may be a tissue section, an individual cell, a cell culture, a spheroid, or an organoid, for example.

In an embodiment of the label the backbone comprises a second nucleic acid strand. This enables increasing the rigidity of the backbone. In particular, the first and second nucleic acid strands may be of a similar or the same length.

In an embodiment of the label the backbone comprises between 20 and 600 nucleotides, in particular between 50 and 250 nucleotides. In case the backbone comprises the first and the second nucleic acid strands, each of the first and the second nucleic acid strands may comprise an essentially equal number of the nucleotides, in particular, such that the first and the second nucleic acid strands can hybridise to form a duplex structure.

In an embodiment of the label the backbone may comprise, in particular consist essentially of, at least one of a natural nucleic acid and a nucleic acid analogue. In particular, the backbone may consist essentially of a nucleic acid analogue. This may render the backbone resistant to degradation agents such as nucleases.

Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxy-ribose, and one of four nucleobases. An analogue may have any of these altered. The nucleic acid analogue may be an artificial nucleic acid or a xeno nucleic acid. Whereas natural nucleic acids or naturally occurring nucleic acids (DNA and RNA) are generally sensitive to degradation agents such as nucleases, nucleic acid analogues are generally resistant to degradation agents such as nucleases.

In a particular embodiment of the label, the at least one hydrophilic moiety is attached to the second nucleic acid strand. This enables efficiently generating the label by initially attaching the at least one labelling moiety to the first nucleic acid strand and attaching the at least one hydrophilic moiety to the second nucleic acid strand before combining the first and second nucleic acid strand to form the backbone of the label.

In the alternative, the at least one hydrophilic moiety may be attached to the first nucleic acid strand.

In an embodiment of the label the first nucleic acid strand and the second nucleic acid strand are configured to form at least one duplex structure. This enables increasing the rigidity of the backbone. In particular, the persistence length of the backbone may be increased. In particular, the increased rigidity of the backbone may enable bright emission of the attached labelling moieties with their native optical properties. For example, the first and second nucleic acid strands may have at least partially complementary sequences with complementary parts of the first and second nucleic acid strands forming the at least one duplex structure.

In a particular embodiment of the label the at least one labelling moiety and/or the at least one hydrophilic moiety is attached to the at least one duplex structure. In particular, the at least one labelling moiety and/or the at least one hydrophilic moiety may be attached to the part of the respective first or second nucleic acid strand that forms the at least one duplex structure. This enables arranging the at least one labelling moiety and/or the at least one hydrophilic moiety at that part of the backbone with the greatest rigidity.

In an embodiment of the label the at least one hydrophilic moiety comprises at least one of a polyethylene glycol (-PEG), a sulfonyl hydroxide (-SO3H), a sulfonate (-SO3), a hydroxyl (-OH), a carboxyl (-COOH), an amine (-NH2), a phosphonate (-PO3H2), a phosphate (-PO4), a carbohydrate (mono-, di-, oligosaccharide), a hyaluronic acid (-HA), or a derivative of any of the aforementioned. This enables efficiently increasing the hydrophilicity of the label. The use of polyethylene glycol as the at least one hydrophilic moiety may be preferred, in particular due to the comparably bulky PEG also reducing unspecific binding of the label due to steric hinderance.

Generally, in an aqueous solution hydrophobic labelling moieties have a tendency to aggregate. Additionally, hydrophobic labelling moieties may cause the label to unspecifically bind to random structures of the biological sample. In particular, hydrophobic labelling moieties that are arranged in close proximity on a flexible single stranded DNA backbone may lead to formation of aggregates. This aggregation changes the optical properties of these labelling moieties such as their absorption wavelength and/or in particular their fluorescence brightness, compared to non-aggregated hydrophobic labelling moieties. By providing the label with the hydrophobic labelling moieties, the nucleic acid backbone having a duplex structure avoids the aggregation of the labelling moieties and therefore enables use of the hydrophobic labelling moieties at their native optical properties. By providing the label with hydrophilic moieties, the label may overall be hydrophilic and therefore avoid binding to structures of the biological sample.

Examples of hydrophobic labelling moieties that are regularly used to analyse biological samples include ATTO 390, ATTO 425, ATTO 550, ATTO Rho12, ATTO 633, and ATTO 647N.

In contrast, hydrophilic labelling moieties may include ATTO 488, and ATTO 532, and ATTO 565 for example.

In an embodiment the label comprises at least five labelling moieties and/or at least five hydrophilic moieties. The at least five labelling moieties may all have the same optical properties or they may have different optical properties. Similarly, the at least five hydrophilic moieties may all be the same or they may be different.

In a particular embodiment, the labelling moieties and/or the hydrophilic moieties are evenly distributed along the backbone.

In an embodiment of the label the at least one labelling moiety and the at least one hydrophilic moiety are arranged along the backbone in an alternating order, in particular, in case the label comprises a plurality of labelling moieties and a plurality of hydrophilic moieties.

In another aspect, a set of labels is provided comprising a plurality of labels as described herein. Each label of the set of labels comprises a first nucleic acid strand with at least one labelling moiety, wherein the at least one labelling moiety of each label has different optical properties, in particular compared to the at least one labelling moiety of the remaining labels of the set of labels. In particular, each label comprises a second nucleic acid strand and each first nucleic acid strand and second nucleic acid strand form a duplex structure based on the same at least partially complementary sequences. Thus, each label may be generated using identical second nucleic acid strands comprising the hydrophilic moieties. This enables efficiently generating the set of labels.

Further, when each label of the set of labels comprises a plurality of labelling moieties, at least one of the plurality of labelling moieties of each label may have different optical properties from other labels. Thus, the combination of labelling moieties of each label of the set of labels may be unique in the set of labels. This enables each label of the set of labels being optically distinguishable from the other labels of the set.

The set of labels enables efficiently analysing a biological sample with a large number of target analytes, in particular distinguishing or identifying a large number of different target analytes.

In a further aspect a marker for analysing a biological sample is provided. The marker comprises a label as described herein, and an affinity reagent configured to specifically bind to a target analyte, in particular of a plurality of target analytes, of the biological sample. The label is attached to the affinity reagent, in particular covalently or via a barcode oligonucleotide. The barcode oligonucleotide of the affinity reagent may be complementary to either the first or second nucleic acid strand.

In a further aspect a set of markers may be provided comprising a plurality of markers as described herein. Each marker of the set of markers comprises one of the labels of the set of labels and an affinity reagent. Each affinity reagent is configured to specifically bind to a different target analyte. A label with unique optical properties is attached to the affinity reagent configured to specifically bind to a particular one of the target analytes.

The affinity reagent may be an antibody, an antibody fragment, or an aptamer, in particular a nucleic acid based aptamer, for example. The affinity reagent enables specifically binding the label to a particular target analyte in the biological sample.

The set of markers enables efficiently analysing a biological sample with a large number of target analytes, in particular distinguishing or identifying a large number of different target analytes.

In a further aspect a method for detecting target analytes of a biological sample is provided. The method comprising incubating the biological sample in the presence of at least one marker as described herein. The method further comprises acquiring a readout of the at least one marker and the biological sample. Based on the readout the presence and/or location of at least one of the target analytes of the biological sample may be determined, in particular, the target analyte to which the affinity reagent of the marker is configured to specifically bind to.

The readout may be an optical readout, for example. In particular, the readout may be acquired by means of a microscope, in particular by means of a light microscope, a fluorescence microscope, a confocal laser scanning microscope, or by means of a cytometer.

In particular, the biological sample may be incubated in the presence of the set of markers, each marker of the set being specific to a particular target analyte. This enables determining the identity or the presence of the respective target analytes in the biological sample.

The method for detecting target analytes of a biological sample and the marker have the same advantages as the label. Further, the method and marker may be supplemented with the features of the label described in this document, in particular, the features of the dependent claims of the label.

In a further aspect a method for generating a label as described herein is provided. The method comprises providing the following: a backbone comprising at least a first nucleic acid strand, at least one optically detectable labelling moiety, and at least one hydrophilic moiety. The at least one labelling moiety and the at least one hydrophilic moiety each comprise a (chemical) reaction group configured to react only under a catalytic condition with respective backbone reaction groups of the backbone to form covalent bonds between the backbone and the at least one labelling moiety and the at least one hydrophilic moiety. The method further comprises applying the catalytic condition to the backbone, the at least one labelling moiety, and the at least one hydrophilic moiety in order to (covalently) attach the at least one labelling moiety and the at least one hydrophilic moiety to the backbone. This generates the label.

In the initial step of providing components of the label a suitable amount of the listed starting materials may be provided, depending on the efficiency of coupling reactions between the respective reaction groups.

The backbone may comprise a plurality of backbone reaction groups, in particular at least two. Depending on the efficiency of coupling reactions between the respective reaction groups, a suitable amount of backbone reaction groups may be provided on the backbone. Additionally, an excess of labelling moieties and/or hydrophilic moieties may be provided. In particular, each reaction group of a labelling moiety and hydrophilic moiety may react with one of the backbone reaction groups to form a covalent bond and attach the respective moiety to the backbone.

Each of the backbone reaction groups may be covalently attached to the backbone, in particular to the respective nucleic acid strand of the backbone. Likewise, the reaction groups of each labelling moiety and hydrophilic moiety may be covalently attached to the respective labelling moiety or hydrophilic moiety.

Backbone reaction groups and reaction groups (of the labelling moieties and hydrophilic moieties) may react with one group of the respective other group. Thus, the backbone reaction groups and reaction groups may form pairs. Examples of such pairs of backbone reaction groups and reaction groups are: click chemistry groups, groups having a phosphoramidite chemistry (these may also be used to incorporate functional groups into DNA/XNA oligonucleotides, i.e. the backbones of the label), groups having an amide bond formation chemistry (also known as "Aminolink" Biomers, these are available with different linker lengths), thiol-maleimide coupling groups (via -SH groups that may be conjugated to nucleic acids using linkers of varying length, also known as "Thiolink" Biomers, these are available with different linker lengths), carboxy groups, aldehyde groups (may be reacted with amino- or thio- groups, hydrazine, hy-drazide, amino oxy acetic acid), cyanobenozothiazol (CBT) and aminothiols like cysteine, which yields luciferin.

In a particular embodiment of the method during the step of providing, the backbone is provided comprising a second nucleic acid strand, wherein both the first nucleic acid strand and the second nucleic acid strand are provided comprising backbone reaction groups of the plurality of backbone reaction groups, in particular each of the backbone reaction groups may be attached to one of the first and second nucleic acid strand. Further, the first nucleic acid strand and the at least one labelling moiety are provided separately from the second nucleic acid strand and the at least one hydrophilic moiety, and in the step of applying the catalytic condition, the catalytic condition is applied to the first nucleic acid strand with the at least one labelling moiety separately to the second nucleic acid strand with the at least one hydrophilic moiety. This enables efficiently attaching the labelling moieties and hydrophilic moieties to specific parts of the label and therefore efficiently generate the label. In particular, the first nucleic acid strand and the at least one labelling moiety are provided physically separated from the second nucleic acid strand and the at least one hydrophilic moiety. For example, the reaction between the backbone reaction groups and the respective reaction groups of the labelling moieties and the hydrophilic moieties may be carried out in separate reaction vessels.

In a particular embodiment of the method, the plurality of backbone reaction groups comprises first reaction groups and second reaction groups, wherein in the step of providing, the first nucleic acid strand is provided comprising the first reaction groups and the second nucleic acid strand is provided comprising the second reaction groups, in particular the first and second reaction groups are attached to the respective nucleic acid strand. The first reaction groups are configured to react under a catalytic condition only with the reaction group of the at least one labelling moiety and the second reaction groups are configured to react under a catalytic condition only with the reaction group of the at least one hydrophilic moiety. This enables efficiently attaching the labelling moieties and hydrophilic moieties to specific parts of the label and therefore efficiently generate the label. In particular, the catalytic conditions may be the same or different for the first and second reaction groups. In this embodiment, the respective catalytic conditions may be applied to the first and second nucleic acid strand, the labelling moiety, and the hydrophilic moiety simultaneously/in same reaction vessel or separately/in separate reaction vessels. Nevertheless, covalent bonds are formed only between the first nucleic acid strand and the labelling moieties and between the second nucleic acid strand and the hydrophilic moieties.

In a further aspect, a system for generating a label is provided. The system comprises a backbone comprising at least a first nucleic acid strand and a plurality of backbone reaction groups, at least one optically detectable labelling moiety, and at least one hydrophilic moiety, wherein the at least one labelling moiety and the at least one hydrophilic moiety each comprise a (chemical) reaction group configured to react (only) under a catalytic condition with backbone reaction groups of the plurality of backbone reaction groups to form covalent bonds between the backbone and the at least one labelling moiety and the at least one hydrophilic moiety. Therefore, this system includes the components of the label. These components may be provided as input for the method for generating a label, in particular as described above.

The system has the same advantages as the method for generating a label and the label. Further, the system and the method may be supplemented with the features of the label described in this document, in particular, the features of the dependent claims of the label.

### Short Description of the Figures

Hereinafter, specific embodiments are described referring to the drawings, wherein:
- Fig. 1: is a schematic view of a first label and an affinity reagent,
- Fig. 2: is a schematic view of a second label and the affinity reagent,
- Fig. 3: is a schematic view of individual components of the first label, and
- Fig. 4: is a workflow of a method for generating a label.

### Detailed Description

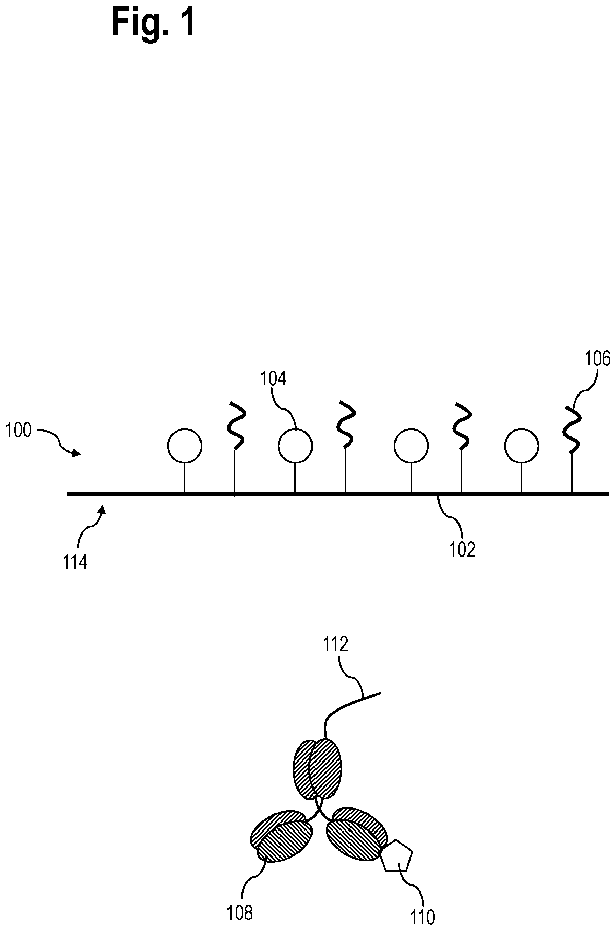

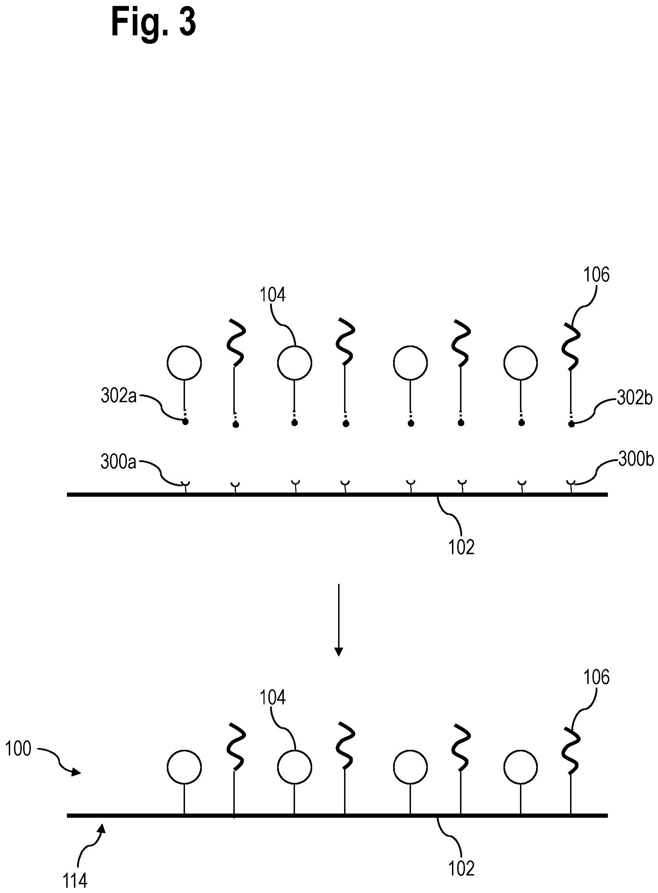

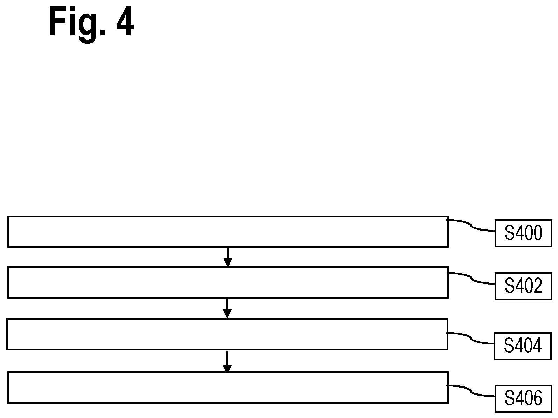

Figure 1 is a schematic view of a first label 100 for analysing a biological sample (not shown). The label 100 comprises a backbone with a first nucleic acid strand 102, to which several labelling moieties 104 and several hydrophilic moieties 106 are attached. The labelling moieties 104 and the hydrophilic moieties 106 are preferably covalently attached to the nucleic acid strand 102. An exemplary way to assemble the label 100 is discussed below with reference to Figs. 3 and 4. The label 100 may have at least one labelling moiety 104 and at least one hydrophilic moiety 106.

The labelling moieties 104 may be fluorophores, for example. Thus, the labelling moieties may be optically detectable, for example, based on their fluorescent excitation and/or emission wavelength, and/or their fluorescent lifetime. The labelling moieties 104 of label 100 all have the same optical properties, such as excitation and emission wavelength and emission lifetime.

Alternatively, the label 100 may have labelling moieties 104 with different optical properties. In case the combination of the optical properties of the labelling moieties 104 of the label 100 is unique within a set of different labels, the label 100 can be identified based on the unique combination of optical properties of its labelling moieties 104.

The hydrophilic moieties 106 may comprise a hydrophilic chemical group, such as a polyethylene glycol chain or an oligosaccharide chain. Further examples of hydrophilic moieties include sulfonyl hydroxides, sulfonates, hydroxyls, carboxyls, amines, phosphonates, phosphates, carbohydrates, and hyaluronic acids. The hydrophilic moieties 106 may all be chemically identical or they may be different.

The hydrophilic moieties 106 increase the hydrophilicity of the label 100. In particular, the hydrophilic moieties 106 may be configured to decrease the hydrophobicity and/or increase the hydrophilicity of the backbone and accordingly of the label 100.

In particular, the hydrophilic moieties 106 is hydrophilic under physiological conditions and/or in an aqueous solution. Thus, the hydrophilic moieties 106 may be hydrophilic when used during analysis of a biological sample.

In particular, by reducing the hydrophobicity of the label 100, the hydrophilic moieties 106 reduce the risk of the label 100 inadvertently and unspecifically binding to random structure of the biological sample.

The label 100 may optionally be attached to an affinity reagent 108, such as an antibody. The affinity reagent 108 is configured to specifically bind to a target analyte 110 of the biological sample, for example a target protein of interest.

In particular, the affinity reagent 108 may comprise a barcode oligonucleotide 112, which is at least partially complementary to a part of the first nucleic acid strand 102, for example, a part of the strand 102 towards a first end 114 of the first nucleic acid strand 102. Alternatively, the affinity reagent 108 may be covalently attached to the label 100.

When the label 100 is attached to the affinity reagent 108 a marker comprising the label 100 and the affinity reagent 108 is generated. The marker may be used to analyse a biological sample. For example, the marker may be used to identify and/or localise the target analyte 110 in the biological sample.

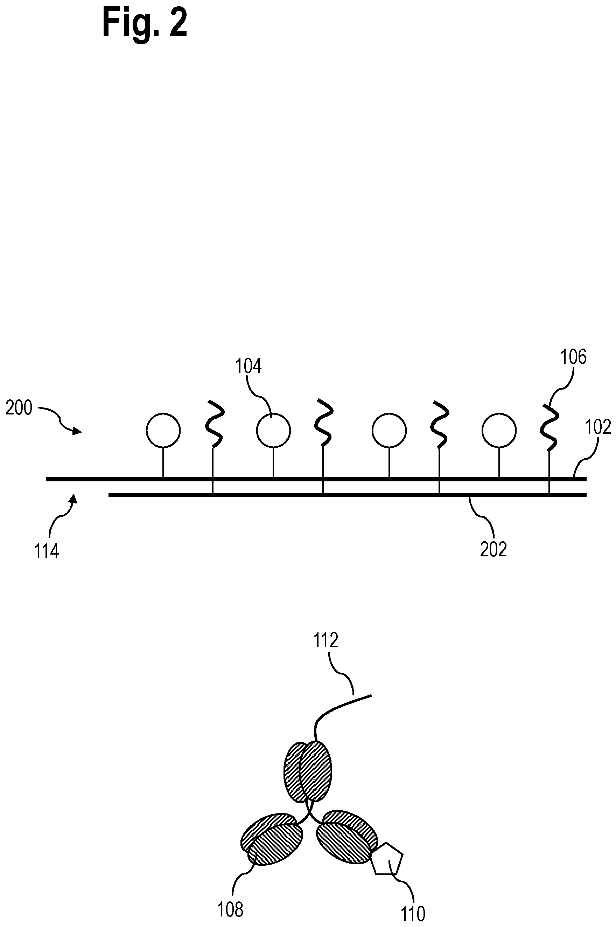

Figure 2 is a schematic view of a second label 200 for analysing a biological sample. The label 200 comprises a backbone with the first nucleic acid strand 102 and a second nucleic acid strand 202. The label 200 further comprises several of the labelling moieties 104 and several of the hydrophilic moieties 106. The labelling moieties 104 are attached, preferably covalently, to the first nucleic acid strand 102 and the hydrophilic moieties 106 are attached, preferably covalently, to the second nucleic acid strand 202. An exemplary way to assemble the label 200 is discussed below with reference to Fig. 4.

The label 200 may have at least one labelling moiety 104 and at least one hydrophilic moiety 106. The labelling moieties 104 of label 200 all have the same optical properties, such as excitation and emission wavelength and emission lifetime.

Alternatively, the label 200 may have labelling moieties 104 with different optical properties. In case the combination of the optical properties of the labelling moieties 104 of the label 200 is unique within a set of different labels, the label 200 can be identified based on the unique combination of optical properties of its labelling moieties 104.

The first and second nucleic acid strands 102, 202 may be at least partially complementary. Thus, the first and second nucleic strands 102, 202 may at least partially hybridise to each other to form a duplex. Thus, the first and second nucleic acid strands 102, 202 may be configured to form a duplex structure.

The labelling moieties 104 and the hydrophilic moieties 106 are preferably attached to parts of the respective first and second nucleic acid strands 102, 202 that form the duplex structure.

In addition to the hydrophilic moieties 106 increasing the overall hydrophilicity of the label and thereby reducing or avoiding unspecific binding of the label 200 to structures of the biological sample, the duplex structure of the backbone of the label 200 increases the rigidity of the backbone. This may further reduce or avoid hydrophobic interactions between labelling moieties 104 of the label 200, in particular by increasing a persistence length of the backbone.

Similar to the label 100, the label 200 may optionally be attached to the affinity reagent 108. The affinity reagent 108 is configured to specifically bind to the target analyte 110 of the biological sample.

When the label 200 is attached to the affinity reagent 108 a marker comprising the label 200 and the affinity reagent 108 is generated. The marker may be used to analyse a biological sample. For example, the marker may be used to identify and/or localise the target analyte 110 in the biological sample.

A set of labels may comprise a plurality of the first label 100 and/or the second label 200, for example. The plurality of labels 100, 200 of the set of labels may each have a combination of the labelling moieties 104 with different optical properties. Thus, each label 100, 200 of the set of labels may have a unique combination of labelling moieties 104 based on their optical properties. This enables distinguishing the labels 100, 200 of the set of labels based on their optical properties, in particular the optical properties of their respective combination of labelling moieties 104.

Similarly, a set of markers may comprise a plurality of markers with each marker comprising one of the labels 100, 200 and an affinity reagent 108. In particular, each marker may comprise an affinity reagent configured to specifically bind to a different target analyte of a biological sample. Thus, with the set of markers a biological sample with a plurality of different target analytes may be analysed. In particular, the presence and/or location of the plurality of different target analytes may be determined in the biological sample by means of the set of markers.

The labels 100, 200, in particular their respective markers, may be applied using a method for detecting target analytes of a biological sample. In a first step, the biological sample may be incubated in the presence of the labels 100, 200, or their respective markers, in particular the set of labels or the set of markers. In particular, when incubating the biological sample in the presence of the set of markers, each marker of the set of markers may comprise an affinity reagent configured to specifically bind to a particular one of the target analytes. Further, each marker comprises one of the labels 100, 200. In particular, all markers of the plurality of markers comprising the affinity reagent to specifically bind to the particular one of the target analytes have the same unique combination of labelling moieties 104. This enables (optically) distinguishing the markers of the set of markers for each of the target analytes.

In a second step of the method a readout is acquired of the biological sample with the labels 100, 200, or their respective markers. The readout may be acquired by means of a microscope, for example. Thus, the readout may be an optical readout.

Based on the readout, the presence and/or the location of the target analytes marked by means of the labels 100, 200, or their respective markers, may be determined.

Figure 3 is a schematic view of individual components of the first label 100 and of steps in method to generate the label 100.

In particular, the label 100 may be generated from the first nucleic acid backbone 102 comprising backbone reaction groups 300a, 300b. Additionally, the labelling moieties 104 and the hydrophilic moieties 106 may be provided comprising reaction groups 302a, 302b. The reactions groups 302a, 302b may be configured to react with the backbone reaction groups 300a, 300b. In particular, the reaction groups 302a of the labelling moieties 104 may be configured to specifically react (only) with a first group of backbone reaction groups 300a. Similarly, the reaction groups 302b of the hydrophilic moieties 106 may be configured to specifically react (only) with a second group of backbone reaction groups 300b. This enables attaching labelling moieties 104 and the hydrophilic moieties 106 to the backbone in a deterministic way rather than randomly. In particular, the ratio of labelling moieties 104 to hydrophilic moieties 106 of the generated label 100 may be controlled this way.

Alternatively, the reaction groups 302a, 302b may be configured to react with any of the backbone reaction groups 300a, 300b.

The reaction between the reaction groups 302a, 302b and the respective backbone reaction groups 300a, 300b may preferably be carried out (only) under a catalytic condition. Thus, the catalytic condition is applied to the individual components of the label 100 in order to attach the labelling moieties 104 and the hydrophilic moieties 106 to the first nucleic acid strand 102 and to generate the label 100.

The reaction between the reaction groups 302a, 302b and the respective backbone reaction groups 300a, 300b may preferably result in a covalent bond between the first nucleic acid strand 102 and the respective labelling moiety 104 or hydrophilic moiety 106.

The individual components of the label 100 may also be termed a system to generate the label 100. Namely, the system may comprise the first nucleic acid strand 102 comprising a plurality of backbone reaction groups 300a, 300b, the labelling moieties 104 comprising reaction groups 302a, and the hydrophilic moieties comprising reaction groups 302b.

The label 200 may be generated analogously based on the second nucleic acid strand 202 also comprising backbone reaction groups 300a, 300b.

In particular, the label 200 may be generated from the first nucleic acid strand 102 only comprising the backbone reaction groups 300a that are configured to react with the reaction groups 302a of the labelling moieties 104, and the second nucleic acid strand 202 only comprising the backbone reaction groups 300b that are configured to react with the reaction groups 302b of the hydrophilic moieties 106. In this case the individual components of the label 200 may all be in the same reaction vessel, when the catalytic condition is applied.

Alternatively, the first and second nucleic acid strand 102, 202 may each comprise backbone reaction groups 300a, 300b that are each configured to react with both reaction groups 302a, 302b of the labelling moiety 104 and the hydrophilic moiety 106. In this case, the first nucleic acid strand 102 and the second nucleic acid strand 202 may be provided in separate reaction vessels together with either the labelling moieties 104 or the hydrophilic moieties 106 and the catalytic condition is applied to the separate reaction vessels. This enables that the labelling moieties 104 and the hydrophilic moieties 106 can nevertheless be attached specifically to either the respective first or the second nucleic acid strands 102, 202.

The backbone reaction groups 300a, 300b and the reaction groups 302a, 302b of the labelling moieties 104 and the hydrophilic moieties 106 may be based on click chemistry, for example. This enables efficiently attaching the labelling moieties 104 and the hydrophilic moieties 106 to the first nucleic acid strand 102 and/or the second nucleic acid strand 202.

Figure 4 is a workflow of a method to generate the label 100, 200. The method starts in a step S400. In step S402 the following are provided: a backbone comprising at least the first nucleic acid strand 102, at least one of the optically detectable labelling moieties 104, and at least one of the hydrophilic moieties 106. The at least one labelling moiety 104 and the at least one hydrophilic moiety 106 each comprise one of the reaction groups 302a, 302b configured to react under a catalytic condition with one of the respective backbone reaction groups 300a, 300b of the backbone to form covalent bonds between the backbone, in particular the first nucleic acid strand 102, and the at least one labelling moiety 104 and the at least one hydrophilic moiety 106.

Optionally, in step S402 the second nucleic acid strand 202 may be provided as part of the backbone.

In step S404 the catalytic condition is applied to the backbone, the at least one labelling moiety 104, and the at least one hydrophilic moiety 106 in order to attach the at least one labelling moiety 104 and the at least one hydrophilic moiety 106 to the backbone, in particular to the first nucleic acid strand 102 and/or to the second nucleic acid strand 202.

The method ends in step S406.

In particular, in case the first and second nucleic acid strands 102, 202 are provided in step S402, these may be provided either in the same or in separate reaction vessels. In case the first and second nucleic acid strands 102, 202 are provided in the same reaction vessel, the first nucleic acid strand 102 may only be provided comprising the backbone reaction groups 300a configured to only react with the reaction groups 302a of the labelling moieties 104 and the second nucleic acid strand 202 may only be provided comprising the backbone reaction groups 300b configured to only react with the reaction groups 302b of the hydrophilic moieties 106. Thus, when the step S404 is subsequently carried out the labelling moieties 104 and the hydrophilic moieties 106 are attached to either the first or the second nucleic acid strand 102, 202.

In case the first and second nucleic acid strands 102, 202 are provided in separate reaction vessels, the first and second nucleic acid strands 102, 202 may be provided comprising the backbone reaction groups 300a, 300b configured to react with both the reaction groups 302a, 302b of the labelling moieties 104 and the hydrophilic moieties 106. In particular, in this case the backbone reaction groups 300a, 300b may be the same and the reaction groups 302a, 302b may be the same.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100, 200: Label
- 102: First nucleic acid strand
- 104: Labelling moiety
- 106: Hydrophilic moiety
- 108: Affinity reagent
- 110: Target analyte
- 112: Barcode oligonucleotide
- 114: First end of first nucleic acid strand
- 202: Second nucleic acid strand
- 300a, 300b: Backbone reaction group
- 302a, 302b: Reaction group of labelling moiety or hydrophilic moiety

## Claims

1. A label (100, 200) for analysing a biological sample comprising:
a backbone comprising at least a first nucleic acid strand (102),
at least one optically detectable labelling moiety (104) attached to the first nucleic acid strand (102), and
at least one hydrophilic moiety (106) attached to the backbone.

2. The label according to claim 1, wherein the backbone comprises a second nucleic acid strand (202).

3. The label according to claim 2, wherein the at least one hydrophilic moiety (106) is attached to the second nucleic acid strand (202).

4. The label according to one of the preceding claims 2 and 3, wherein the first nucleic acid strand (102) and the second nucleic acid strand (202) are configured to form at least one duplex structure.

5. The label according to claim 4, wherein the at least one labelling moiety (104) and/or the at least one hydrophilic moiety (106) is attached to the at least one duplex structure.

6. The label according to one of the preceding claims, wherein the at least one hydrophilic moiety (106) comprises at least one of a polyethylene glycol, a sulfonyl hydroxide, a sulfonate, a hydroxyl group, a carboxyl group, an amine, a phosphonate, a phosphate, a carbohydrate, and a hyaluronic acid.

7. The label according to one of the preceding claims comprising at least five labelling moieties (104) and/or at least five hydrophilic moieties (106).

8. The label according to one of the preceding claims, wherein the at least one labelling moiety (104) and the at least one hydrophilic moiety (106) are arranged along the backbone in an alternating order.

9. A set of labels comprising a plurality of labels (100, 200) according to one of the preceding claims, in particular according to at least claim 2, wherein each label (100, 200) of the set of labels comprises a first nucleic acid strand (104) with at least one labelling moiety (104), wherein the at least one labelling moiety (104) of each label has different optical properties, and in particular wherein each label comprises a second nucleic acid strand (202) and each first nucleic acid strand (102) and second nucleic acid strand (202) form a duplex structure based on the same at least partially complementary sequences.

10. A marker for analysing a biological sample comprising
a label (100, 200) according to one of the preceding claims 1 to 8, and
an affinity reagent (108) configured to specifically bind to a target analyte (110) of the biological sample,
wherein the label (100, 200) is attached to the affinity reagent (108).

11. A method for detecting target analytes (110) of a biological sample, the method comprising the following steps:
incubating the biological sample in the presence of at least one marker according to claim 10, and
acquiring a readout of the at least one marker and the biological sample.

12. A method for generating a label (100, 200) according to one of the preceding claims 1 to 8, the method comprising the following steps:
providing the following: a backbone comprising at least a first nucleic acid strand (102), at least one optically detectable labelling moiety (104), and at least one hydrophilic moiety (106), wherein the at least one labelling moiety (104) and the at least one hydrophilic moiety (106) each comprise a reaction group (302a, 302b) configured to react under a catalytic condition with respective backbone reaction groups (300a, 300b) of the backbone to form covalent bonds between the backbone and the at least one labelling moiety (104) and the at least one hydrophilic moiety (106), and
applying the catalytic condition to the backbone, the at least one labelling moiety (104), and the at least one hydrophilic moiety (106) in order to attach the at least one labelling moiety (104) and the at least one hydrophilic moiety (106) to the backbone.

13. The method according to claim 12, wherein in the step of providing, the backbone is provided comprising a second nucleic acid strand (202), wherein both the first nucleic acid strand (102) and the second nucleic acid strand (202) are provided comprising backbone reaction groups (300a, 300b) of the plurality of backbone reaction groups (300a, 300b), wherein the first nucleic acid strand (102) and the at least one labelling moiety (104) are provided separately from the second nucleic acid strand (202) and the at least one hydrophilic moiety (106), and wherein in the step of applying the catalytic condition, the catalytic condition is applied to the first nucleic acid strand (102) with the at least one labelling moiety (104) separately to the second nucleic acid strand (202) with the at least one hydrophilic moiety (106).

14. The method according to claim 12, wherein the plurality of backbone reaction groups (300a, 300b) comprises first reaction groups (300a) and second reaction groups (300b), wherein in the step of providing, the first nucleic acid strand (102) is provided comprising the first reaction groups (300a) and the second nucleic acid strand (202) is provided comprising the second reaction groups (300b), wherein the first reaction groups (300a) are configured to react under a catalytic condition only with the reaction group (302a) of the at least one labelling moiety (104) and the second reaction groups (300b) are configured to react under a catalytic condition only with the reaction group (302b) of the at least one hydrophilic moiety (106).
